# EUROPEAN PATENT SPECIFICATION

(11) **EP 1 117 693 B1**
(45) Date of publication and mention of the grant of the patent: **17.05.2006**
(21) Application number: 99950063.0
(22) Date of filing: 30.09.1999
(51) Int. Cl.: C07K 19/00, C12N 15/62, C12P 21/02, C12P 21/04, C12N 9/90

(54) **INTEIN MEDIATED PEPTIDE LIGATION**
INTEIN VERMITTELTE PEPTIDLIGATION
LIGATION DE PEPTIDE INDUITE PAR UNE INTEINE

(30) Priority: 30.09.1998 US 102413 P
(43) Date of publication of application: 25.07.2001
(73) Proprietor: NEW ENGLAND BIOLABS, INC., Beverly Massachusetts 01915 (US)
(72) Inventor: XU, Ming-Qun, Hamilton, MA 01982 (US); EVANS, Thomas, C., Somerville, MA 02143 (US)
(74) Representative: Froud, Clive
(86) International application number: PCT/US1999/022776
(87) International publication number: WO 2000/018881

(56) References cited:
- US-A- 5 834 247
- SEVERINOV K ET AL: "Expressed protein ligation, a novel method for studying protein-protein interactions in transcription." THE JOURNAL OF BIOLOGICAL CHEMISTRY. UNITED STATES 26 JUN 1998, vol. 273, no. 26, 26 June 1998 (1998-06-26), pages 16205-16209, XP002238975 ISSN: 0021-9258
- XU M-Q ET AL: "THE MECHANISM OF PROTEIN SPLICING AND ITS MODULATION BY MUTATION" EMBO JOURNAL, OXFORD UNIVERSITY PRESS, SURREY, GB, vol. 15, no. 19, 1 October 1996 (1996-10-01), pages 5146-5153, XP001063196 ISSN: 0261-4189
- LYKKE-ANDERSEN J ET AL: "The C-terminal carboxy group of T7 RNA polymerase ensures efficient magnesium ion-dependent catalysis." NUCLEIC ACIDS RESEARCH. ENGLAND 15 DEC 1998, vol. 26, no. 24, 15 December 1998 (1998-12-15), pages 5630-5635, XP002238976 ISSN: 0305-1048
- SOUTHWORTH M W ET AL: "Purification of proteins fused to either the amino or carboxy terminus of the Mycobacterium xenopi gyrase A intein." BIOTECHNIQUES. UNITED STATES JUL 1999, vol. 27, no. 1, July 1999 (1999-07), pages 110-114, 116, 118 - 120, XP001147377 ISSN: 0736-6205
- DAWSON P E ET AL: "Synthesis of proteins by native chemical ligation." SCIENCE. UNITED STATES 4 NOV 1994, vol. 266, no. 5186, 4 November 1994 (1994-11-04), pages 776-779, XP000889577 ISSN: 0036-8075
- CHONG S. ET AL.: 'Single-column purification of free recombinant proteins using a self-cleavable affinity tag derived from a protein splicing element' GENE vol. 192, no. 2, 19 June 1997, pages 271 - 281, XP002925637
- EVANS T.C. ET AL.: 'Semisynthesis of cytotoxic proteins using a modified protein splicing element' PROTEIN SCIENCE vol. 7, no. 11, 05 November 1998, pages 2256 - 2264, XP002925638
- MUIR T.W. ET AL.: 'Expressed protein ligation: A general method for protein engineering' PROC. NATL. ACAD. SCI. USA vol. 95, June 1998, pages 6705 - 6710, XP002925639
- EVANS T.C. ET AL.: 'The Cyclization and Polymerization of Bacterially Expressed Proteins Using Modified Self-splicing Inteins' THE JOURNAL OF BIOLOGICAL CHEMISTRY vol. 274, no. 26, June 1999, pages 19359 - 18363, XP002925640
- TELENTI A. ET AL.: 'The mycobacterium xenopi GyrA Protein Splicing Element: Characterization of a Minimal Intein' JOURNAL OF BACTERIOLOGY vol. 179, no. 20, 14 October 1997, pages 6378 - 6382, XP002925641

## Description

### BACKGROUND OF THE INVENTION

Genetic engineering is a powerful approach to the manipulation of proteins. However, genetic methodologies are constrained by the use of only naturally coded amino acids. Furthermore, cytotoxic proteins are difficult to obtain by expression and isolation from a living source, since the expression of the toxic protein can result in death of the host.

To some extent, protocols have been developed to circumvent these problems, for example, total chemical synthesis (Kent, S. B. (1988) *Ann. Rev. Biochem.* 57:957-989), use of misacylated tRNAs (Noren, et al., (1989) *Science* 244:182-188), and semi-synthetic techniques (reviewed in Offord, R. (1987) *Protein Eng*. 1:151-157; Roy. et al. (1994) *Methods in Enzymol*. 231:194-215; Wallace, C. J. (1993) *FASEB* 7:505-515). However, all of these procedures are limited by either the size of the fragment which can be generated or by low reaction yield.

It would therefore be desirable to develop a high-yield, semi-synthetic technique to allow *in vitro* fusion of a synthetic protein or peptide fragment to an expressed protein without limitation as to the size of the fused fragments.

Likewise, in order to produce cytotoxic proteins, it would be desirable to develop a method of fusing a synthetic fragment, *in vitro*, to an inactive, expressed protein, so as to restore protein activity post-production from the host.

The modified Sce VMA intein has been used to generate thioester-tagged proteins for use in ligation (Example 19, U.S.S.N. 08/811,492, filed June 16, 1997; Chong, (1996) *J. Biol. Chem.,* 271 (36):22159-22168; Chong, (1997) Gene, 192:271-281; and Muir, et al. (1998) *Proc. Natl. Acad. Sci* USA 95:6705-6710).

Some disadvantages have been low yields due to poor cleavage of the Sce VMA intein with thiol-reagents that are optimum for ligation, the need for large peptide quantities due to on-column reactions, the use of odoriferous reagents, and/or low protein yields due to the use of a large, eukaryotic intein.

### SUMMARY OF THE INVENTION

In general terms, a method has now been found for producing a semi-synthetic fusion protein *in vitro*, comprising the steps of producing a target protein fused to a protein splicing element (an intein) and selectively cleaving the fusion and ligating a synthetic protein or peptide at the C-terminal thioester of the target protein, which overcome many of the disadvantages and problems noted above. Such a method has higher yields due to better thiol-induced cleavage with thiol reagents which have been optimized for the ligation reaction. Off-column ligation allows for sample concentration, as well as the use of less peptide. In a particularly preferred embodiment, the thiol reagent 2-mercaptoethanesulfonic acid (MESNA), which is an odorless thiol-reagent, is used for cleavage and ligation along with the Mxe intein, which is from a bacterial source and often expresses better in bacterial cells. Furthermore, the new approach allows peptides to be directly ligated to the thioester bond formed between an intein and the target protein. A method has also been found for producing a cytotoxic protein, comprising the steps of producing a truncated, inactive form of the protein *in vivo* which is fused to a protein splicing element, and selectively cleaving the fusion and ligating a synthetic protein or peptide at a C-terminal thioester of the target protein to restore the activity of the native cytotoxic protein. Recombinant vectors for producing such cleavable fusion proteins are also disclosed.

### BRIEF DESCRIPTION OF THE DRAWINGS

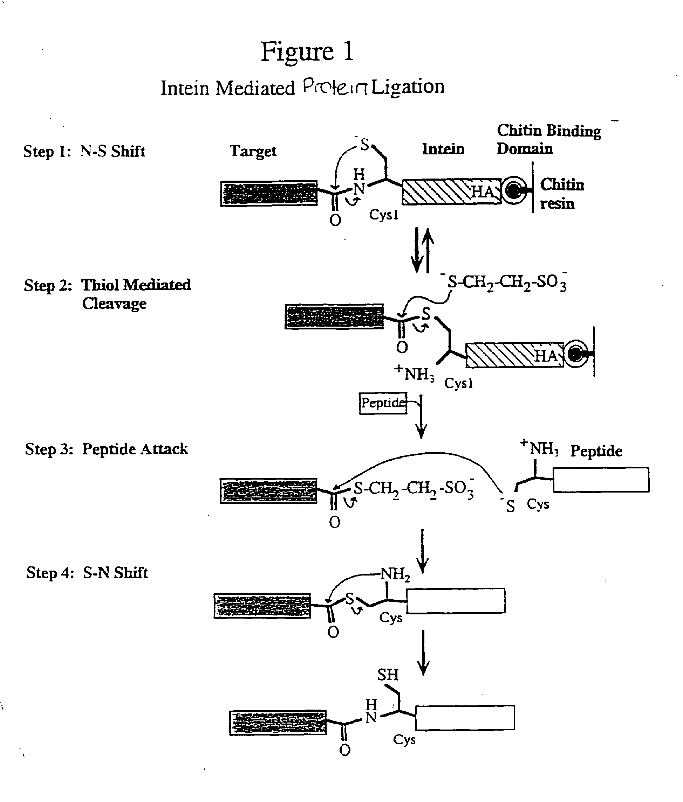

Figure 1 is a flow diagram depicting the chemical reactions which enable intein-mediated peptide ligation. The thioester generated at the C-terminus of the target protein during IMPACT™ purification was used in a 'native chemical ligation' reaction. This allowed the ligation of a synthetic peptide to a bacterially expressed protein. A typical ligation - reaction involved the expression of the target protein-intein-CBD fusion followed by binding to a chitin resin. A thiol reagent induced cleavage of the intein. The target was eluted from the chitin resin and a synthetic peptide was added. The ligation reaction proceeded overnight.

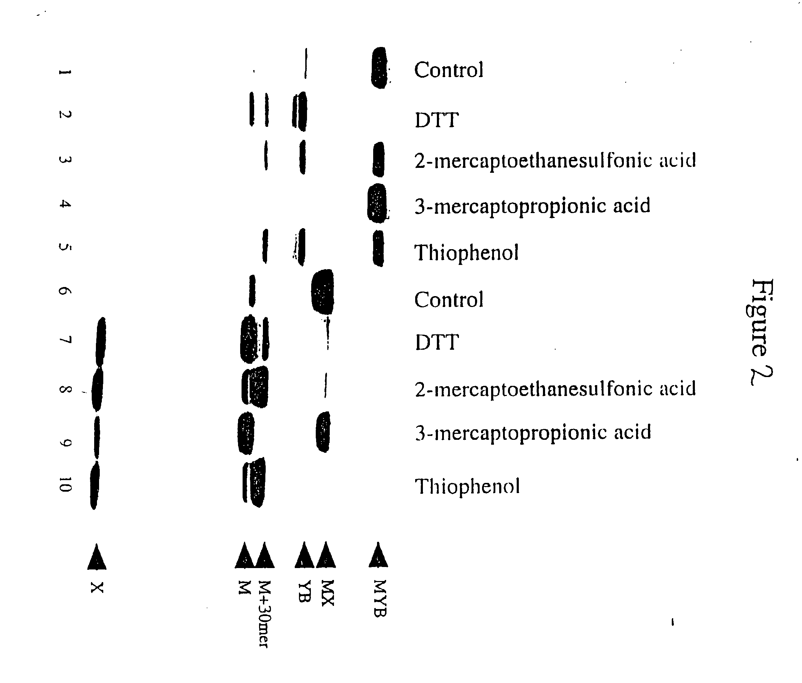

Figure 2 is a gel depicting the results of cleavage and ligation reactions using various thiols. Cleavage and ligation reactions with different thiols visualized on 10-20% Tricine gels. MYB (a fusion protein of maltose binding protein-Sce VMA intein (N454A)-chitin binding domain) and MXB (a fusion protein of maltose binding protein-Mxe GyrA (N198A) intein-chitin binding domain) were incubated overnight at 4°C with various thiols (50 mM) in 150 mM Tris, 100 mM NaCl, pH 8 in the presence of a 30 amino acid peptide with an N-terminal cysteine. The peptide ligates to the C-terminus of MBP. Lanes 1-5 ligation with MYB. Lane 1 no thiol. Lane 2 dithiothreitol. Lane 3 2-mercaptoethanesulfonic acid. Lane 4 3-mercaptopropionic acid. Lane 5 thiophenol. Lanes 6-10 ligation with MXB. Lane 6 no thiol. Lane 7 dithiothreitol. Lane 8 2-mercaptoethanesulfonic acid. Lane 9 3-mercaptopropionic acid. Lane 10 thiophenol.

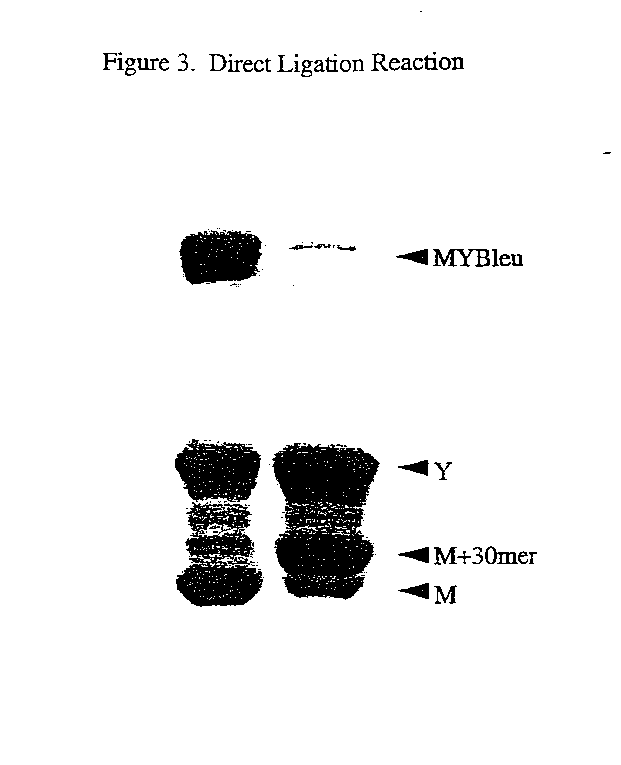

Figure 3 is a gel depicting direct ligation of a peptide to the thioester formed between the Sce VMA intein and maltose binding protein. SDS-PAGE of direct ligation reaction with a 10-20% Tricine gel. Lane 1: a precursor protein (MYBleu) - consisting of maltose binding protein-Sce VMA1 intein-chitin binding domain was heated to >95°C for 5 minutes in a buffer of 50 mM Trizma base, pH 8.5 containing 100 mM NaCl, 1% SDS, and mM tris-(2-carboxyethyl)phosphine (TCEP) followed by overnight incubation at room temperature. The precursor (MYBIeu) is visible along with the Sce VMA1 intein (Y) and maltose binding protein (M), which are cleavage products. Lane 2: the precursor protein was subjected to the same conditions as described in Lane 1 except that the 30 amino acid peptide (1 mM) was added. The precursor (MYB) and cleavage products (Y and M) are visible along with the ligation product (M+30mer) formed when the 30 amino acid peptide fuses to maltose binding protein.

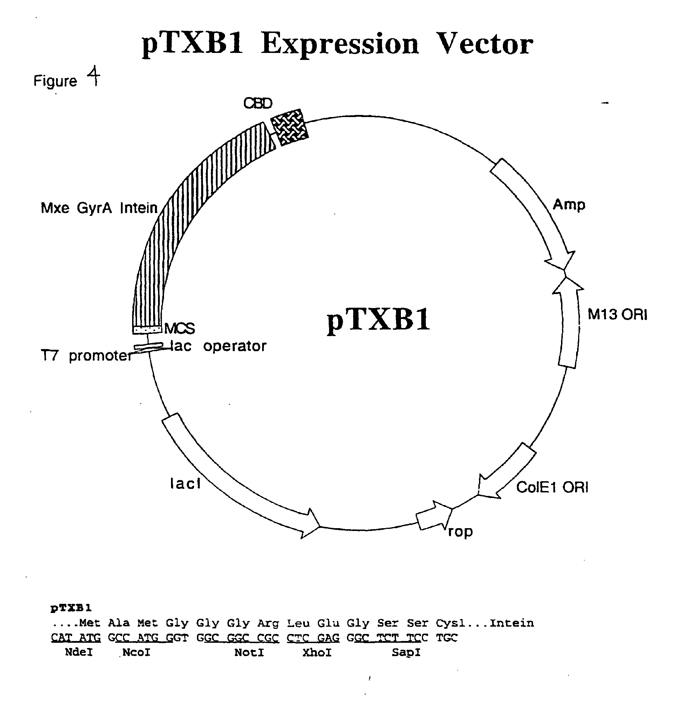

Figure 4 is a diagram depicting the pTXB1 expression vector of Example I (SEQ ID NO:7 and SEQ ID NO:8).

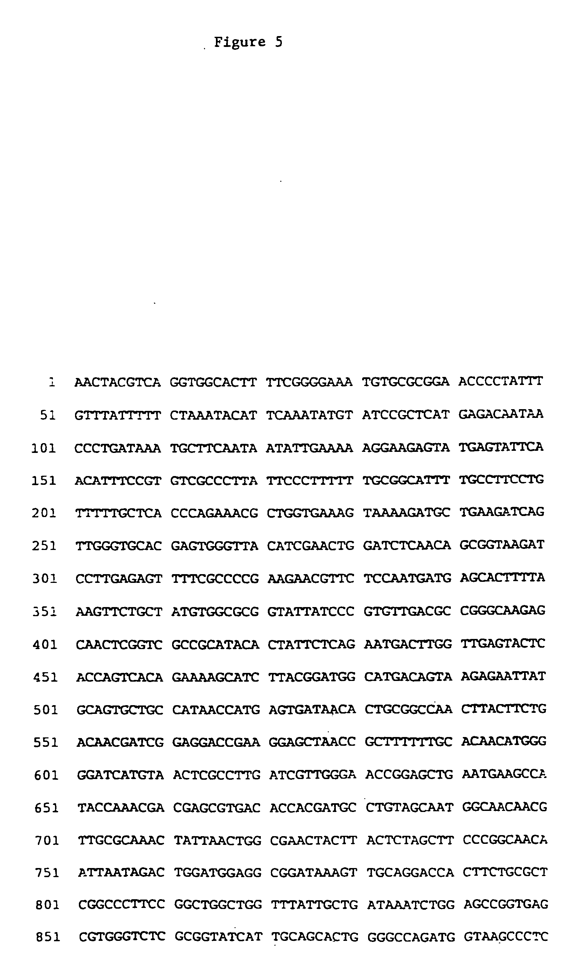

Figure 5 is the DNA sequence of pTXB1 (SEQ ID NO:5).

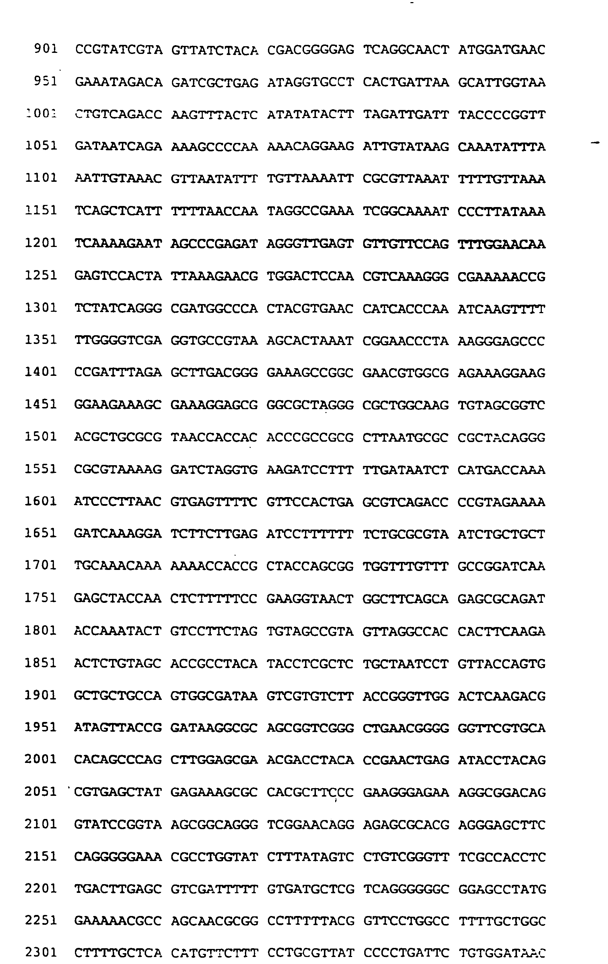

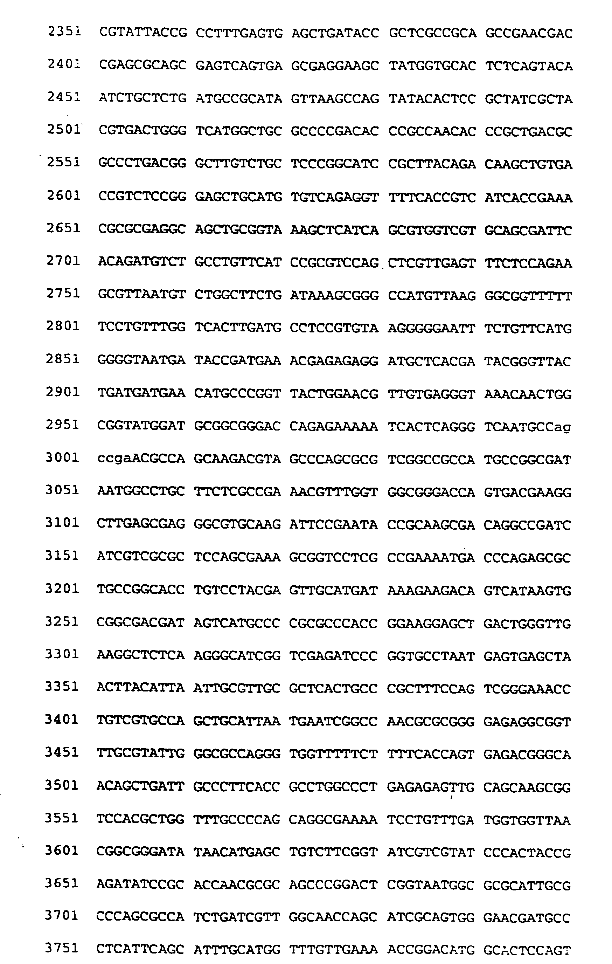

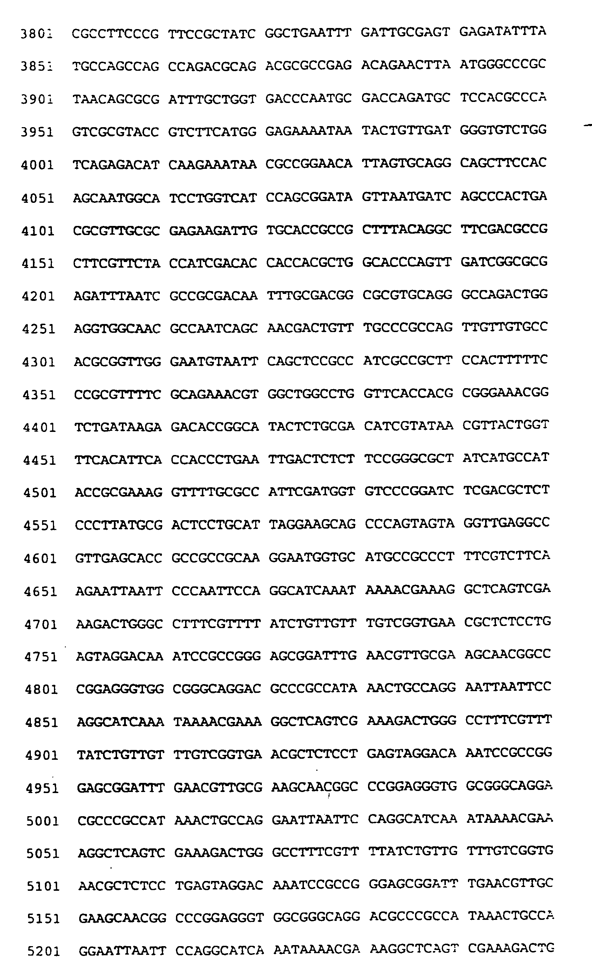

Figure 6 is a gel depicting the results of the *Hpa*I protein ligation reaction. Protein ligation reactions examined on 10-20% Tricine gels. Lane 1: clarified cells extract after I PTG (0.5 mM) induction of ER2566 cells containing the pTXB2-*Hpa*I plasmid. The fusion protein of *Hpa*I₂₂₃-MXE GyrA-intein-CBD (52 kDa) is visible. Lane 2: cell extract as in Lane 1 after passage over a chitin column, which results in the binding of the fusion protein. Lane 3: *Hpa*I₂₂₃ (25.7 kDa) after cleavage from the fusion protein by addition of MESNA. Lane 4: ligation product of *Hpa*I₂₂₃ (0.2 mg/mL) with 1 mM of a 31 amino acid peptide (ligation product 29.6 kDa), representing the residues necessary to generate full length *Hpa*I, after overnight incubation at 4 °C. Lane 5: full length *Hpa*I from a recombinant source (29.6 kDa) containing BSA (66 kDa) and two impurities.

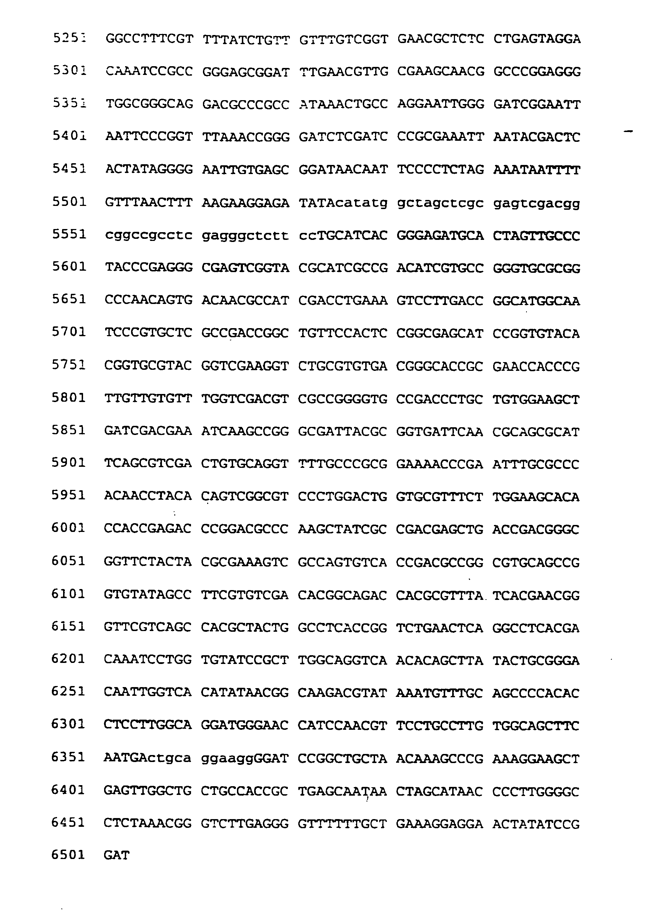

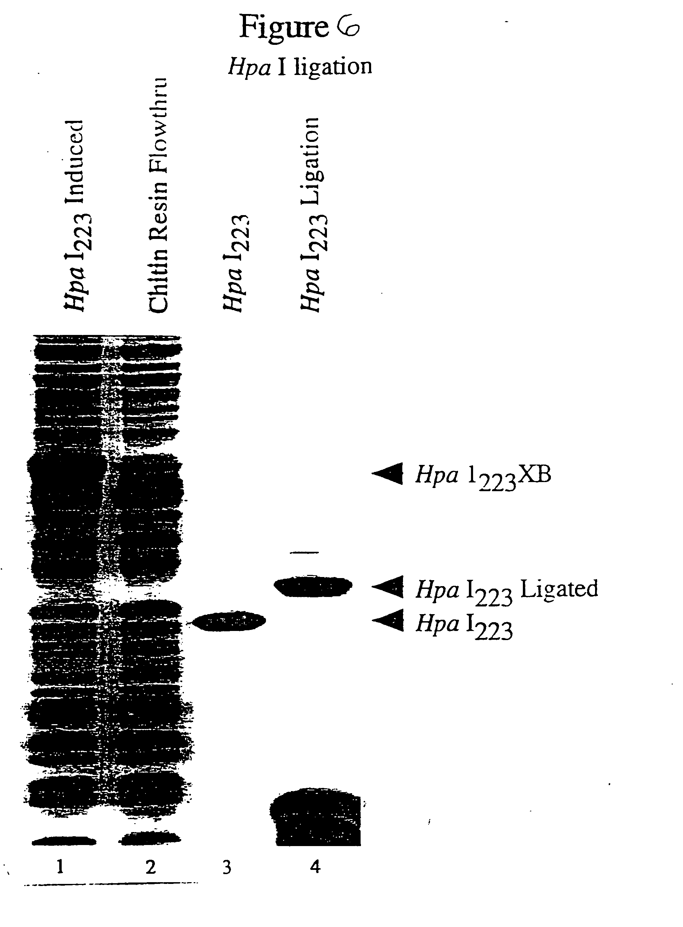

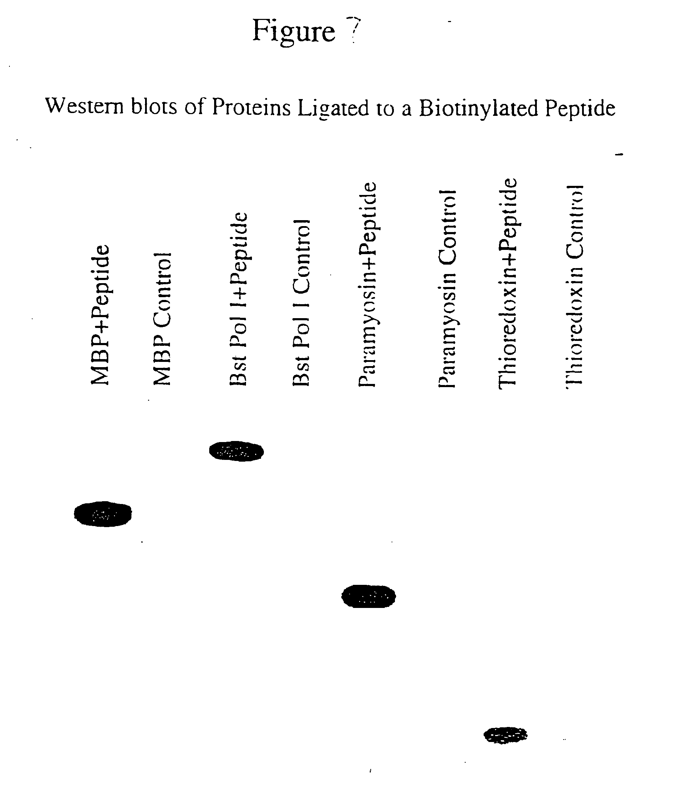

Figure 7 is a westem blot of various proteins ligated to a biotinylated peptide. Proteins purified with the Mxe GyrA IMPACT™ derivative were ligated to a synthetic peptide which contained an antibody recognition sequence.

### DETAILED DESCRIPTION OF THE INVENTION

In one embodiment, the present invention provides a method for preparing a target protein with a carboxy-terminal thioester suitable for peptide ligation, characterised in that the method comprises:
(a) expressing, in a host cell, a recombinant precursor protein comprising the target protein fused at its carboxy terminus to an intein, the intein having an amino-terminus and a carboxy-terminus, the amino-terminus of the intein being fused to the target protein, and the intein being selected from a native intein, an intein derivative or an intein mutant;
   and
(b) contacting the expressed precursor protein with 2-mercaptoethanesulfonic acid and inducing cleavage of the intein from the precursor protein so as to form the target protein having the carboxy-terminal thioester.

Preferably, the intein is selected from *Saccharomyces cerevisiae* Vma intein and *Mycobacterium xenopi* Gyr A intein. The carboxy terminus of the intein may be fused to a protein binding domain, which is a chitin binding domain. The target protein may be selected from a *Bacillus stearothermophilus* DNA polymerase I large fragment, thioredoxin or a cytotoxic protein, or from a maltose binding protein and paramyosin.

In another embodiment, the present invention provides a method for expressing a recombinant protein precursor, characterised in that the method comprises:
(a) inserting a nucleic acid sequence encoding a target protein into a plasmid at a multiple cloning site located upstream of and in frame with a fusion gene encoding an intein and a chitin binding domain, such that
   (i) the intein is selected from a naturally occurring intein, an intein derivative or an intein mutant; and
   (ii) the multiple cloning site is formed from a linker of SEQ ID NO:3 and SEQ ID NO:4;
   and
(b) introducing the plasmid into a host cell and providing conditions suitable for expressing the recombinant precursor protein by the host cell.

In a further embodiment, the present invention provides a method of modifying a target protein by ligating a chemically synthesized peptide or protein *in vitro* to the target protein, characterised in that the method comprises:
(a) expressing, in a host cell, the target protein fused at its carboxy terminus to an intein selected from an intein, an intein derivative or a mutant intein, the intein being capable of thiol induced cleavage;
(b) inducing cleavage of the intein from the target protein by adding 2-mercaptoethanesulfonic acid so as to form a carboxy-terminal thioester on the target protein;
(c) obtaining the chemically synthesized peptide or protein having an amino-terminal cysteine;
   and
(d) ligating the target protein of (b) to the chemically synthesized peptide or protein of (c) to form a modified target protein.

Preferably, the intein is fused at its carboxy terminus to a chitin binding domain. The protein, prior to modification, may be a cytotoxic protein.

In another embodiment, the present invention provides a method of labeling a target protein, characterised in that the method comprises:
(a) expressing, in a host cell, a recombinant precursor protein comprising the target protein fused at its carboxy terminus to an intein, the intein having an amino and a carboxy terminus such that the intein is fused at the amino terminus to the target protein, the intein being selected from a naturally occurring intein, an intein derivative or an intein mutant, and the intein being capable of thiol induced cleavage;
(b) cleaving the precursor protein in the presence of 2-mercaptoethanesulfonic acid so as to form the target protein having a carboxy-terminal thioester;
(c) obtaining a chemically synthesized peptide or protein having a marker and an amino-terminal cysteine;
   and
(d) ligating the target protein of (b) with the chemically synthesized peptide or protein of (c) for labeling the target protein.

Preferably, the intein is fused at its carboxy terminus to a chitin binding domain. The marker may be selected from a fluorescent marker, a spin label, an affinity tag or a radiolabel. The chemically synthesized peptide or protein is an antigenic determinant.

In a further embodiment, the present invention provides a method for ligating a chemically synthesized protein or peptide to an inactive form of a protein so as to restore protein activity, characterised in that the method comprises:
(a) expressing, in a host cell, a fusion protein comprising the inactive form of the protein fused at its carboxy-terminus to one of an intein, an intein derivative or an intein mutant, the fusion protein being expressed from a plasmid;
(b) inducing intein mediated cleavage of the protein of (a) by adding 2-mercaptoethanesulfonic acid so as to form a carboxy-terminal thioester on the inactive protein;
(c) obtaining a chemically synthesized protein or peptide having an amino-terminal cysteine;
   and
(d) ligating the inactive protein of (b) to the chemically synthesized peptide or protein of (c) to restore protein activity.

The protein may be a cytotoxic protein, which may be a restriction endonuclease.

Having indicated the scope of the present invention, it will now be further described and illustrated in more general terms.

The ligation methods of the present invention are based on the discovery that a cysteine or peptide fragment containing an N-terminal cysteine may be fused, *in vitro*, to a bacterially expressed protein produced by thiol-induced cleavage of an intein (U.S. Patent No. 5,496,714; Example 19 of U.S.S.N. 08/811,492 filed June 16, 1997; Chong, et al., (1996) *supra* and Chong, et al., (1997) *supra.*

The ligation procedure disclosed herein utilizes a protein splicing element, an intein (Perler, et al., (1994) *Nucleic Acids Res.* 22:1125-1127) to precisely create a thioester at the C-terminal α-carbon of an expressed protein. This reactive thioester could be present between the target protein and intein or generated by the addition of a thiol reagent. Previously the generation such a thioester was described using an intein (CIVPS) that was modified to undergo thiol inducible cleavage at its N-terminal junction in the presence of thiol reagent dithiothreitol (DTT) (Chong, et al. (1997) *supra;* Comb, et.al. U.S. Patent No. 5,496,714). This C-terminal thioester was previously used in a 'native chemical ligation type reaction (Dawson, et al., (1994) *Science* 266:776-779) to fuse ³⁵S-cysteine or a peptide fragment containing an N-terminal cysteine to a bacterially expressed protein (Example 19, Comb, et.al. U.S. Patent No. 5,834,247, Chong (1996) *supra* and Chong (1997) *supra.*

The ligation method of the instant invention begins with the purification of the thioester-tagged target protein using an intein as described (Chong, et.al. (1997) *supra).* The direct ligation method of the instant invention begins with the isolation of a precursor composed of the target protein-intein-CBD. In one preferred embodiment, the host cell is bacterial. In other embodiments the host cell may be yeast, insect, or mammalian. A cysteine thiol at the N-terminus of a synthetic peptide nucleophilicly attacks a thioester present on the freshly isolated C-terminal α-carbon of the target protein or directly attacks the thioester present between the target protein and intein. This initially generates a thioester between the two reactants which spontaneously rearranges - into a native peptide bond (Figure 1).

In order to optimize the ligation efficiency so that greater than 90% of the bacterially expressed target protein can be fused to the synthetic peptide or protein, specific thiol reagents and inteins are screened. In a preferred embodiment, the intein may be any CIVPS, such as *Sce* VMA, *Mxe* GyrA or derivatives of mutants thereof, and the thiol reagent is 2-mercapto-ethanesulfonic acid, thiophenol, DTT, or 3-mercaptopropionic acid (Comb, et al., U.S. Patent No. 5,496,714; U.S. Patent No. 5,834,247).

In one particularly preferred embodiment, an intein whose protein splicing activity has been blocked by mutation is utilized. The mutant must, however, retain the ability to undergo the N-S shift, thus allowing thioester formation between itself and an N-terminal protein. This thioester can then be nucleophilicly attacked by a thiol reagent or by the N-terminal cysteine of a peptide sequence. For example, by mutating the C-terminal asparagine (asn 198) of an intein from the GyrA gene of *Mycobacterium xenopi* (Telenti, et al., (1997) *J Bacteriol* 179:6378-6382) to an alanine created a thiol inducible cleavage element. This modified intein cleaved well with thiol reagents that were optimal for the ligation reaction, such as MESNA and thiophenol. Furthermore, optimal thiol reagent and intein combinations can be determined by - incubating a precursor protein containing the intein of interest with a wide variety of thiol reagents followed by determination of the extent of cleavage of the precursor protein (Figure 2).

The use of such intein and specific thiol reagents leads to optimal yields and high ligation efficiencies; typically greater than 90% of the N-terminal ligation fragment can be modified.

The ligation methods of the present invention expand the ability to incorporate non-coded amino acids into large protein sequences by generating a synthetic peptide fragment with fluorescent probes, spin labels, affinity tags, radiolabels, or antigenic determinants and ligating this to an *in vivo* expresed protein isolated using a modified intein.

Furthermore, this procedure allows the isolation of cytotoxic proteins by purifying an inactive truncated precursor from a host source, for example bacteria, and generating an active protein or enzyme after the ligation of a synthetic peptide. For example, restriction endonucleases which have not successfully been cloned by traditional methods may be produced in accordance with the present invention.

Also, the direct ligation procedure allows the ligation of - a protein or peptide sequence to another protein or peptide sequence without the use of exogenous thiol reagents. Direct ligation relies on the nucleophilic attack of the N-terminal amino acid of one peptide on the thioester formed between a target protein and an intein (Figure 3).

In summary, a fusion protein can be created using the methods of the present invention that possesses unique properties which, currently, can not be generated genetically.

The Examples presented below are only intended as specific preferred embodiments of the present invention and are not intended to limit the scope of the invention. The present invention encompasses modifications and variations of the methods taught herein which would be obvious to one of ordinary skill in the art.

The references cited above and below are herein incorporated by reference.

### EXAMPLE I

### Creation of vectors pTXB1 and pTXB2 for ligation:

Asparagine 198 of the *Mxe* GyrA intein (Telenti, et al., (1997) *J Bacteriol*. 179:6378-6382) was mutated to alanine by linker insertion into the *Xmn*I and *Pst*I sites of pmxeMIPTyrXmnSPdel to create pMXP1. The *Xmn*l site was originally introduced into the unmodified *Mxe* GyrA intein sequence by silent mutagenesis. The *Pst*I site was a unique site in the plasmid. The linker was composed of mxe#3 (5'-GGTTCGTCAGCCACGCTACTGGCCTCACCGGTTGATAGCTGCA-3') (SEQ ID NO:1) and mxe#4 (5'-GCTATCAACCGGTGAGGCCAGTAG CGTGGCTGACGAACC-3') (SEQ ID NO:2).

Into pMXP1 another linker composed of mxe#1 (5'-TC GAATCTAGACATATGGCCATGGGTGGCGGCCGCCTCGAGGGCTCTTCC TGCATCACGGGAGATGCA-3') (SEQ ID NO:3) and mxe#2 (5'-CTAG TGCATCTCCCGTGATGCAGGAAGAGCCCTCGAGGCGHGCCGCCACCCA TGGCCATATGTCTAGAT-3') (SEQ ID NO:4) was inserted into the *Xho*I and *Spe*I sites to introduce a multiple cloning site (*Xba*I-*Nde*I-*Nco*I-*Not*I-*Xho*I-*Sap*I) before the *Mxe* GyrA intein (pMXP2).

The 0.6 kilobase *Not*I to *Age*I fragment of pMXP2 was ligated into the same sites in pTYB1 (IMPACT kit, New England Biolabs, Beverly, MA) and the *Nco*I to *Age*I fragment of pMXP2 was cloned into pTYB3 (IMPACT kit, New England Biolabs, Beverly, MA) to create plasmids pTXB1 (see Figure 4 and 5) (SEQ ID NO:5) and pTXB2, respectively. These vectors have a multiple cloning site upstream of the modified *Mxe* GyrA intein-chitin binding domain fusion. This allows the insertion of a target gene of interest inframe with the intein and chitin binding domain (CBD).

### Creation of vectors pMYBleu for ligation:

pMYBleu was as described in Chong, et al., (1998), *J. Biol*. *Chem.* 273:10567-10577. This vector consisted of maltose binding protein upstream of the Sce VMA intein-chitin binding domain. A leucine is present at the -1 position instead of the native residue (which is a glycine).

### Purification of Thioester-Tagged Proteins:

Protein purification was as described using the *Sce* VMA intein (Chong, et.al., (1997) *Gene* 192:271-281) with slight modification. ER2566 cells (IMPACT T7 instruction manual from New England Biolabs, Beverly, MA) containing the pTXB vector with the appropriate insert were grown to an OD₆₀₀ of 0.5-0.6 at 37°C at which point they were induced with 0.5 mM IPTG overnight at 15°C. Cells were harvested by centrifugation and lysed by sonication (performed on ice). The three part fusion protein was bound to chitin beads (10 mL bed volume, Figure 6, lanes 1 and 2) equilibrated in Buffer A (50 mM Tris, pH 7.4, and 500 mM NaCl), and washed with 10 column volumes of Buffer A to remove unbound material.

Cleavage was initiated using a buffer of 50 mM 2-mercaptoethanesulfonic acid (MESNA), 50 mM Tris, pH 8.0 and 100 mM NaCl. Other thiol reagents were also used at other times, such as thiophenol, dithiothreitol, and/or 3-mercaptopropionic acid. After overnight incubation at from 4-25°C protein was eluted from the column (Figure 6 lane 3). This protein contained a thioester at the C-terminus.

### Purification of MYB. MYBleu and MXB:

Full length precursor proteins consisting of maltose binding protein-*Sce* VMA intein (N454A)-chitin binding domain (MYB) and maltose binding protein-*Mxe* GyrA (N198A) intein-chitin binding domain (MXB) were purified after induction and sonication, as described above, by applying the sonicated sample to a 10 mL column of amylose resin (New England Biolabs, Beverly, MA). Unbound proteins were washed from the column with 10 column volumes of Buffer A (see purification of thioester-tagged proteins). Bound proteins were eluted with a buffer of 50 mM Tris, pH 8, containing 100 mM NaCl and 10 mM maltose. Fractions were collected and protein concentrations were determined using the Bio-Rad Protein Assay (Hercules, CA).

### Peptide Synthesis:

Peptides for subsequent ligation reactions were synthesized on an ABI model 433A peptide synthesizer utilizing *FastMoc*™ chemistry (Fields, et al., (1991) *Pept Res* 4, 95-101) at a 0.085 mmol scale. Preloaded HMP (p-hydroxymethylphenoxymethyl) polystyrene resins (Applied Biosystems, Foster City, CA) functionalized at 0.5 mmol/g was used in conjunction with Fmoc/NMP chemistry utilizing HBTU amino acid activation (Dourtoglou, et al., (1984) *Synthesis* 572-574; Knorr, et al., (1989) *Tetrahedron Lett* 30, 1927-1930). Fmoc amino acids were purchased from Applied Biosystems (Foster City, CA).

Synthesis proceeded with a single coupling during each cycle. Peptide cleavage from the resin and simultaneous removal of side chain protecting groups was facilitated by the addition of cleavage mixture (Perkin Elmer, Norwalk, CT) consisting of 0.75 g phenol, 0.25 mL 1,2-ethanedithiol, 0.5 mL deionized H₂O, and 10 mL TFA. The resin was flushed with nitrogen and gently stirred at room temperature for 3 hours. Following filtration and precipitation into cold (0°C) methyl-t-butyl ether, the precipitate in the ether fraction was collected by centrifugation. The peptide precipitate was vacuum dried and analyzed by mass spectrometry using a Perceptive Biosystems (Framingham, MA) MALDI-TOF mass spectrometer.

Final purification was by HPLC using a Waters HPLC system with a Lambda-Max Model 481 Multiwavelength detector (set at 214 nm), 500 series pumps and automated gradient controller with a Vydac semi-preparative C18 column. Elution of the peptide was with a 60 minute linear gradient of 6-60% acetonitrile (v/v) in an aqueous solution of 0.1% TFA (v/v).

### Protein Cleavage and Ligation Reactions:

Cleavage of MYB and MXB: The precursor protein (1 mg/mL) was incubated overnight at 4°C with or without a thiol reagent (50 mM) in 150 mM Tris, pH 8, containing 100 mM NaCl.

Ligation reactions with MYB and MXB: The precursor protein (1 mg/mL) was treated as described for cleavage except that a 30 amino acid peptide (1 mM final concentration, NH₂-CAYKTTQANKHIIVACEGNPYVPVHFDASV-COOH (SEQ ID NO:6) was also included in the reaction (Figure 2).

Ligation reactions after purification of thioester-tagged proteins: Lyophilized peptides (New England Biolabs, Beverly, MA) were added (to 1 mM final concentration) directly to the thioester-tagged protein freshly isolated from the chitin column. The reaction was allowed to proceed overnight at from 4-25°C. In both ligation procedures the condensation of the reactants is visible on a 10-20% Tricine gel (Figure 6). The ligation reaction was tested in conditions of 5-150 mM Tris or HEPES buffers, 50-1000 mM NaCl, 10 mM Maltose, and pH 6-11 and 0-6 M Urea.

### Direct Ligation Reactions:

MYBleu (1 mg/mL) was incubated in 6 M Urea or 1% SDS, pH 7.5-8.5, 50-200 mM NaCl, and 1 mM of a 30 amino acid peptide (NH₂CAYKTTQANKHIVVACEGNPYVPVHFDASV-COOH (SEQ ID NO:6)). The MYBleu was incubated for 0-180 minutes at either 4°C or 100°C prior to the addition of the 30 amino acid peptide. Ligation reactions proceeded overnight at either 4°C or 25°C.

### EXAMPLE II

### Labeling a target protein: Maltose Binding Protein

Maltose binding protein (MBP, 42 kDa) was isolated as described in Example I above using the IMPACT procedure (IMPACT manual from New England Biolabs, Inc., Beverly, MA) in the presence of MESNA.

A biotinylated peptide possessing an N-terminal cysteine (CDPEK*DS-COOH (SEQ ID NO:9)), in which the biotin was attached to the ε-amino group of the lysine residue) was ligated to the freshly purified target protein as described above. Briefly, 4 µL of biotinylated peptide (10 mM) were mixed with a 36 µL aliquot of the freshly purified MBP sample. The mixture was incubated at 4°C overnight.

Western blots with alkaline phosphatase linked anti-biotin antibody detected the presence of the ligated product but not the unligated target protein (Figure 7). The efficiency of the ligation is typically greater than 90% when MESNA is used for cleavage.

### EXAMPLE III

### Labeling a target protein: Bst DNA Polymerase I Large Fragment (Bst Pol 1)

Bst DNA Polymerase I large fragment (67 kDa) was isolated as described in Example I above using the IMPACT procedure (IMPACT manual from New England Biolabs, Inc., Beverly, MA) in the presence of MESNA.

A biotinylated peptide possessing an N-terminal cysteine (CDPEK*DS-COOH (SEQ ID NO:9)), in which the biotin was attached to the ε-amino group of the lysine residue) was ligated to the freshly purified target protein as described. Briefly, 4 µL of biotinylated peptide (10 mM) were mixed with a 36 µL aliquot of the freshly purified Bst Pol 1 sample. The mixture was incubated at 4°C overnight.

Western blots with alkaline phosphatase linked anti-biotin antibody detected the presence of the ligated product but not the unligated target protein (Figure 7). The efficiency of the ligation is typically greater than 90% when MESNA is used for cleavage.

### EXAMPLE IV

### Labeling a target protein: Paramyosin

Paramyosin (29 kDa) was isolated as described in Example I above using the IMPACT procedure (IMPACT manual from New England Biolabs, Inc., Beverly, MA) in the presence of MESNA.

A biotinylated peptide possessing an N-terminal cysteine (CDPEK*DS-COOH (SEQ ID NO:9)), in which the biotin was attached to the ε-amino group of the lysine residue) was ligated to the freshly purified target protein as described. Briefly, 4 µL of biotinylated peptide (10 mM) were mixed with a 36 µL aliquot of the freshly purified paramyosin sample. The mixture was incubated at 4°C overnight.

Western blots with alkaline phosphatase linked anti-biotin antibody detected the presence of the ligated product but not the unligated target protein (Figure 7). The efficiency of the ligation is typically greater than 90% when MESNA is used for cleavage.

### EXAMPLE V

### Labeling a target protein: E. coli Thioredoxin

*E. coli* thioredoxin (12 kDa) was isolated as described in Example I above using the IMPACT procedure (IMPACT manual from New England Biolabs, Inc., Beverly, MA) in the presence of MESNA.

A biotinylated peptide possessing an N-terminal cysteine (CDPEK*DS-COOH (SEQ ID NO:9)), in which the biotin was attached to the ε-amino group of the lysine residue) was ligated to the freshly purified target protein as described. Briefly, 4 µL of biotinylated peptide (10 mM) were mixed with a 36 µL aliquot of the freshly purified thioredoxin sample. The mixture was incubated at 4°C overnight.

Western blots with alkaline phosphatase linked anti-biotin antibody detected the presence of the ligated product but not the unligated target protein (Figure 7). The efficiency of the ligation is typically greater than 90% when MESNA is used for cleavage.

### EXAMPLE VI

### Isolation of a cytotoxic protein:

The ligation procedure of Example I was applied to the isolation of a potentially cytotoxic protein. An endonuclease from *Haemophilus parainfluenzae (Hpa*I Ito, et al., (1992) *Nucleic Acids Res* 20:705-709) was generated by ligating an inactive truncated form of the enzyme expressed in *E. coli* (ER2566 cells, New England Biolabs, Inc., Beverly, MA) with the missing amino acids that were synthesized chemically.

The first 223 amino acids of *Hpa*I (full length *Hpa*I is 254 amino acids) were fused in frame with the modified *Mxe* GyrA intein and the CBD. The 223 amino acid *Hpa*I fragment was isolated as described for purification of thioester tagged proteins. The truncated *Hpa*I displayed no detectable enzymatic activity.

A synthetic peptide representing the 31 amino acids needed to complete *Hpa*I was ligated onto the 223 amino acid truncated form of *Hpa*I by the method of Example I.

### Enzymatic Assay for HpaI:

The activity of the fused *Hpa*I was determined by its ability to digest Lambda DNA (New England Biolabs, Beverly, MA). Serial dilutions of ligated or truncated *Hpa*I, with the appropriate peptide added to 1 mM, were incubated with 1 µg of Lambda DNA for 1 hour at 37°C in a buffer of 20 mM Tris-acetate, pH 7.9, 10 mM magnesium acetate, 50 mM potassium acetate, 1 mM dithiothreitol, and 170 µg/mL BSA (total volume 30 µL). Digestion reactions were visualized on 1% agarose gels permeated with ethidium bromide. One unit of *Hpa* I was defined as the amount of enzyme necessary to digest 1 µg of Lambda DNA in one hour at 37°C.

The newly ligated *Hpa*I had a specific activity of 0.5-1.5x10⁶ units/mg which correlated well with the expected value of 1-2x10⁶ units/mg for the full length enzyme.

## Claims

1. A method for preparing a target protein with a carboxy-terminal thioester suitable for peptide ligation, **characterised in that** the method comprises:
(a) expressing, in a host cell, a recombinant precursor protein comprising the target protein fused at its carboxy terminus to an intein, the intein having an amino-terminus and a carboxy-terminus, the amino-terminus of the intein being fused to the target protein, and the intein being selected from a native intein, an intein derivative or an intein mutant;
and
(b) contacting the expressed precursor protein with 2-mercaptoethanesulfonic acid and inducing cleavage of the intein from the precursor protein so as to form the target protein having the carboxy-terminal thioester.

2. A method as claimed in claim 1 wherein the intein is selected from *Saccharomyces cerevisiae* Vma intein and *Mycobacterium xenopi* Gyr A intein.

3. A method as claimed in claim 1 wherein the carboxy terminus of the intein is fused to a protein binding domain, which is a chitin binding domain.

4. A method as claimed in claim 1 wherein the target protein is selected from a *Bacillus stearothermophilus* DNA polymerase I large fragment, thioredoxin or a cytotoxic protein.

5. A method as claimed in claim 1 wherein the target protein is selected from a maltose binding protein and paramyosin.

6. A method for expressing a recombinant protein precursor, **characterised in that** the method comprises:
(a) inserting a nucleic acid sequence encoding a target protein into a plasmid at a multiple cloning site located upstream of and in frame with a fusion gene encoding an intein and a chitin binding domain, such that
(i) the intein is selected from a naturally occurring intein, an intein derivative or an intein mutant;
and
(ii) the multiple cloning site is formed from a linker of SEQ ID NO:3 and SEQ ID NO:4;
and
(b) introducing the plasmid into a host cell and providing conditions suitable for expressing the recombinant precursor protein by the host cell.

7. A method of modifying a target protein by ligating a chemically synthesized peptide or protein *in vitro* to the target protein, **characterised in that** the method comprises:
(a) expressing, in a host cell, the target protein fused at its carboxy terminus to an intein selected from an intein, an intein derivative or a mutant intein, the intein being capable of thiol induced cleavage;
(b) inducing cleavage of the intein from the target protein by adding 2-mercaptoethanesulfonic acid so as to form a carboxy-terminal thioester on the target protein;
(c) obtaining the chemically synthesized peptide or protein having an amino-terminal cysteine;
and
(d) ligating the target protein of (b) to the chemically synthesized peptide or protein of (c) to form a modified target protein.

8. A method as claimed in claim 7 wherein the intein is fused at its carboxy terminus to a chitin binding domain.

9. A method as claimed in claim 7 wherein the protein, prior to modification, is a cytotoxic protein.

10. A method of labeling a target protein, **characterised in that** the method comprises:
(a) expressing, in a host cell, a recombinant precursor protein comprising the target protein fused at its carboxy terminus to an intein, the intein having an amino and a carboxy terminus such that the intein is fused at the amino terminus to the target protein, the intein being selected from a naturally occurring intein, an intein derivative or an intein mutant, and the intein being capable of thiol induced cleavage;
(b) cleaving the precursor protein in the presence of 2-mercaptoethanesulfonic acid so as to form the target protein having a carboxy-terminal thioester;
(c) obtaining a chemically synthesized peptide or protein having a marker and an amino-terminal cysteine;
and
(d) ligating the target protein of (b) with the chemically synthesized peptide or protein of (c) for labeling the target protein.

11. A method as claimed in claim 10 wherein the intein is fused at its carboxy terminus to a chitin binding domain.

12. A method as claimed in claim 10 wherein the marker is selected from a fluorescent marker, a spin label, an affinity tag or a radiolabel.

13. A method as claimed in claim 10 wherein the chemically synthesized peptide or protein is an antigenic determinant.

14. A method for ligating a chemically synthesized protein or peptide to an inactive form of a protein so as to restore protein activity, **characterised in that** the method comprises:
(a) expressing, in a host cell, a fusion protein comprising the inactive form of the protein fused at its carboxy-terminus to one of an intein, an intein derivative or an intein mutant, the fusion protein being expressed from a plasmid;
(b) inducing intein mediated cleavage of the protein of (a) by adding 2-mercaptoethanesulfonic acid so as to form a carboxy-terminal thioester on the inactive protein;
(c) obtaining a chemically synthesized protein or peptide having an amino-terminal cysteine;
and
(d) ligating the inactive protein of (b) to the chemically synthesized peptide or protein of (c) to restore protein activity.

15. A method as claimed in claim 14 wherein the protein is a cytotoxic protein.

16. A method as claimed in claim 15 wherein the cytotoxic protein is a restriction endonuclease.

## Patentansprüche

1. Verfahren zur Herstellung eines Targetproteins mit einem carboxy-terminalen Thioester, der zur Peptidligierung geeignet ist, **dadurch gekennzeichnet, dass** das Verfahren umfasst:
a) Exprimieren, in einer Wirtszelle, eines rekombinanten Vorläuferproteins, umfassend das Targetprotein, das an seinem Carboxyterminus an ein Intein fusioniert ist, wobei das Intein einen Aminoterminus und einen Carboxyterminus besitzt, wobei der Aminoterminus des Inteins an das Targetprotein fusioniert ist, und das Intein aus einem nativen Intein, einem Inteinderivat oder einer Inteinmutante ausgewählt wird; und
b) In Kontakt bringen des exprimierten Vorläuferproteins mit 2-Mercaptoethansulfonsäure und Induzieren der Abspaltung des Inteins vom Vorläuferprotein, um das Targetprotein mit dem carboxy-terminalen Thioester zu bilden.

2. Verfahren nach Anspruch 1, wobei das Intein aus *Saccharomyces cerevisiae* Vma-Intein und *Mycobacterium xenopi* Gyr A-Intein ausgewählt wird.

3. Verfahren nach Anspruch 1, wobei der Carboxyterminus des Inteins an eine Proteinbindende Domäne fusioniert wird, die eine Chitin-bindende Domäne ist.

4. Verfahren nach Anspruch 1, wobei das Targetprotein ausgewählt wird aus *Bacillus stearothermophilus* DNA Polymerase I großes Fragment, Thioredoxin oder einem cytotoxischen Protein.

5. Verfahren nach Anspruch 1, wobei das Targetprotein ausgewählt wird aus einem Maltosebindenden Protein und Paramyosin.

6. Verfahren zum Exprimieren eines rekombinanten Proteinvorläufers, **dadurch gekennzeichnet, dass** das Verfahren umfasst:
a) Insertieren einer Nukleinsäuresequenz, die für ein Targetprotein kodiert, in ein Plasmid an einer multiplen Klonierungsstelle, die stromaufwärts von und im Leseraster mit einem Fusionsgen, welches für ein Intein und eine Chitin-bindende Domäne kodiert, lokalisiert ist, so dass
i) das Intein ausgewählt wird aus einem natürlich vorkommenden Intein, einem Inteinderivat oder einer Inteinmutante; und
ii) die multiple Klonierungsstelle durch einen Linker der SEQ ID NO:3 und SEQ ID NO:4 gebildet wird; und
b) Einführen des Plasmids in eine Wirtszelle und Bereitstellen von Bedingungen, die zur Expression des rekombinanten Vorläuferproteins durch die Wirtszelle geeignet sind.

7. Verfahren zum Modifizieren eines Targetproteins durch Ligieren eines chemisch synthetisierten Peptids oder *Proteins in vitro* an das Targetprotein, **dadurch gekennzeichnet, dass** das Verfahren umfasst:
a) Exprimieren, in einer Wirtszelle, das an seinem Carboxyterminus an ein Intein fusionierte Targetprotein, wobei das Intein ausgewählt wird aus einem Intein, einem Inteinderivat oder einer Mutante des Inteins, wobei das Intein zu einer Thiol-induzierten Spaltung befähigt ist;
b) Induzieren der Abspaltung des Inteins aus dem Targetprotein durch Zugabe von 2-Mercaptoethansulfonsäure, um einen carboxy-terminalen Thioester am Targetprotein zu bilden;
c) Erhalten des chemisch synthetisierten Peptids oder Proteins mit einem Amino-terminalen Cystein; und
d) Ligieren des Targetproteins aus b) an das chemisch synthetisierte Peptid oder Protein aus c), um ein modifiziertes Targetprotein zu bilden.

8. Verfahren nach Anspruch 7, wobei das Intein an seinem Carboxyterminus an eine Chitin-bindende Domäne fusioniert wird.

9. Verfahren nach Anspruch 7, wobei das Protein, vor seiner Modifikation, ein cytotoxisches Protein ist.

10. Verfahren zum Markieren eines Targetproteins, **dadurch gekennzeichnet, dass** das Verfahren umfasst:
a) Exprimieren, in einer Wirtszelle, eines rekombinanten Vorläuferproteins, umfassend das Targetprotein, das an seinem Carboxyterminus an ein Intein fusioniert ist, wobei das Intein einen Amino- und einen Carboxyterminus aufweist, so dass das Intein an den Aminoterminus des Targetproteins fusioniert ist, wobei das Intein ausgewählt wird aus einem natürlich vorkommenden Intein, einem Inteinderivat oder einer Inteinmutante, und das Intein zu einer Thiol-induzierten Spaltung befähigt ist;
b) Spalten des Vorläuferproteins durch Zugabe von 2-Mercaptoethansulfonsäure, um das Targetprotein mit einem carboxy-terminalen Thioester zu bilden;
c) Erhalten des chemisch synthetisierten Peptids oder Proteins mit einem Marker und einem Amino-terminalen Cystein; und
d) Ligieren des Targetproteins aus b) mit dem chemisch synthetisierten Peptid oder Protein aus c), um das Targetprotein zu markieren.

11. Verfahren nach Anspruch 10, wobei das Intein an seinem Carboxyterminus an eine Chitin-bindende Domäne fusioniert wird.

12. Verfahren nach Anspruch 10, wobei der Marker ausgewählt wird aus einem fluoreszierenden Marker, einem Spin-Label, einem Affinitäts-Tag oder einer Radiomarkierung.

13. Verfahren nach Anspruch 10, wobei das chemisch synthetisierte Peptid oder Protein eine antigene Determinante ist.

14. Verfahren zum Ligieren eines chemisch synthetisierten Proteins oder Peptids an eine inaktive Form eines Proteins, um die Proteinaktivität wieder herzustellen, **dadurch gekennzeichnet, dass** das Verfahren umfasst:
a) Exprimieren, in einer Wirtszelle, eines Fusionsproteins, welches die inaktive Form des Proteins, fusioniert an seinem Carboxyterminus an ein Intein, ein Inteinderivat oder eine Inteinmutante, umfasst, wobei das Fusionsprotein von einem Plasmid exprimiert wird;
b) Induzieren der durch das Intein vermittelten Spaltung des Proteins aus a) durch Zugabe von 2-Mercaptoethansulfonsäure, um einen carboxy-terminalen Thioester an dem inaktiven Protein zu bilden;
c) Erhalten eines chemisch synthetisierten Proteins oder Peptids mit einem Amino-terminalen Cystein; und
d) Ligieren des inaktiven Proteins von b) an das chemisch synthetisierte Peptid oder Protein von c), um die Proteinaktivität wieder herzustellen.

15. Verfahren nach Anspruch 14, wobei das Protein ein cytotoxisches Protein ist.

16. Verfahren nach Anspruch 15, wobei das cytotoxische Protein eine Restriktionsendonuklease ist.

## Revendications

1. Procédé de préparation d'une protéine cible avec un thioester à terminaison carboxy appropriée pour la ligature peptidique, **caractérisé en ce que** le procédé comprend :
(a) l'expression, dans une cellule hôte, d'une protéine précurseur recombinée comprenant la protéine cible fusionnée au niveau de sa terminaison carboxy à une intéine, l'intéine ayant une terminaison amino et une terminaison carboxy, la terminaison amino de l'intéine étant fusionnée à la protéine cible, et l'intéine étant sélectionnée parmi une intéine native, un dérivé d'intéine ou un mutant d'intéine ;
et
(b) la mise en contact de la protéine précurseur exprimée avec de l'acide 2-mercaptoéthanesulfonique et l'induction du clivage de l'intéine de la protéine précurseur de manière à former la protéine cible ayant le thioester à terminaison carboxy.

2. Procédé selon la revendication 1, dans lequel l'intéine est sélectionnée parmi l'intéine Vma de *Saccharomyces cerevisiae* et l'intéine Gyr A de *Mycobacterium xenopi.*

3. Procédé selon la revendication 1, dans lequel la terminaison carboxy de l'intéine est fusionnée à un domaine de liaison protéique qui est un domaine de liaison à la chitine.

4. Procédé selon la revendication 1, dans lequel la protéine cible est sélectionnée parmi un grand fragment d'ADN polymérase I de *Bacillus stearothermophilus*, la thiorédoxine ou une protéine cytotoxique.

5. Procédé selon la revendication 1, dans lequel la protéine cible est sélectionnée parmi une protéine de liaison au maltose et la paramyosine.

6. Procédé d'expression d'une protéine précurseur recombinée, **caractérisé en ce que** le procédé comprend :
(a) l'insertion d'une séquence d'acides nucléiques codant pour une protéine cible dans un plasmide au niveau d'un site de clonage multiple situé en amont et dans le cadre d'un gène de fusion codant pour une intéine et un domaine de liaison à la chitine, de sorte que
(i) l'intéine est sélectionnée parmi une intéine se produisant naturellement, un dérivé d'intéine ou un mutant d'intéine ;
et
(ii) le site de clonage multiple est formé à partir d'un lieur de SEQ ID N° : 3 et SEQ ID N° : 4 ;
et
(b) l'introduction du plasmide dans une cellule hôte et la fourniture de conditions appropriées pour l'expression de la protéine précurseur recombinée par la cellule hôte.

7. Procédé de modification d'une protéine cible par ligature d'un peptide ou d'une protéine synthétisé(e) de manière chimique in vitro à la protéine cible, **caractérisé en ce que** le procédé comprend :
(a) l'expression, dans une cellule hôte, de la protéine cible fusionnée au niveau de sa terminaison carboxy à une intéine sélectionnée parmi une intéine, un dérivé d'intéine ou un mutant d'intéine, l'intéine étant capable de clivage induit par un thiol ;
(b) l'induction du clivage de l'intéine de la protéine cible par ajout d'acide 2-mercaptoéthanesulfonique de manière à former un thioester à terminaison carboxy sur la protéine cible ;
(c) l'obtention du peptide ou de la protéine synthétisé(e) de manière chimique ayant une cystéine à terminaison amino ;
et
(d) la ligature de la protéine cible de (b) au peptide ou à la protéine synthétisé(e) de manière chimique de (c) pour former une protéine cible modifiée.

8. Procédé selon la revendication 7, dans lequel l'intéine est fusionnée au niveau de sa terminaison carboxy à un domaine de liaison à la chitine.

9. Procédé selon la revendication 7, dans lequel la protéine est une protéine cytotoxique avant la modification.

10. Procédé de marquage d'une protéine cible, **caractérisé en ce que** le procédé comprend :
(a) l'expression, dans une cellule hôte, d'une protéine précurseur recombinée comprenant la protéine cible fusionnée au niveau de sa terminaison carboxy à une intéine, l'intéine ayant une terminaison amino et une terminaison carboxy de sorte que l'intéine est fusionnée au niveau de la terminaison amino à la protéine cible, l'intéine étant sélectionnée parmi une intéine se produisant naturellement, un dérivé d'intéine ou un mutant d'intéine, et l'intéine étant capable de clivage induit par un thiol ;
(b) le clivage de la protéine précurseur en présence d'acide 2-mercaptoéthanesulfonique de manière à former la protéine cible ayant un thioester à terminaison carboxy ;
(c) l'obtention d'un peptide ou d'une protéine synthétisé(e) de manière chimique ayant un marqueur et une cystéine à terminaison amino ;
et
(d) la ligature de la protéine cible de (b) au peptide ou à la protéine synthétisé(e) de manière chimique de (c) pour le marquage de la protéine cible.

11. Procédé selon la revendication 10, dans lequel l'intéine est fusionnée au niveau de sa terminaison carboxy à un domaine de liaison à la chitine.

12. Procédé selon la revendication 10, dans lequel le marqueur est sélectionné parmi un marqueur fluorescent, un marqueur de spin, un marqueur d'affinité ou un radiomarqueur.

13. Procédé selon la revendication 10, dans lequel le peptide ou la protéine synthétisé(e) de manière chimique est un déterminant antigénique.

14. Procédé de ligature d'une protéine ou d'un peptide synthétisé(e) de manière chimique à une forme inactive d'une protéine de manière à restaurer l'activité protéique, **caractérisé en ce que** le procédé comprend :
(a) l'expression, dans une cellule hôte, d'une protéine de fusion comprenant la forme inactive de la protéine fusionnée au niveau de sa terminaison carboxy à l'un parmi une intéine, un dérivé d'intéine ou un mutant d'intéine, la protéine de fusion étant exprimée à partir d'un plasmide ;
(b) l'induction du clivage induit par une intéine de la protéine de (a) par ajout d'acide 2-mercaptoéthanesulfonique de manière à former un thioester à terminaison carboxy sur la protéine inactive ;
(c) l'obtention d'une protéine ou d'un peptide synthétisé(e) de manière chimique ayant une cystéine à terminaison amino ;
et
(d) la ligature de la protéine inactive de (b) au peptide ou à la protéine synthétisé(e) de manière chimique de (c) pour restaurer l'activité protéique.

15. Procédé selon la revendication 14, dans lequel la protéine est une protéine cytotoxique.

16. Procédé selon la revendication 15, dans lequel la protéine cytotoxique est une endonucléase de restriction.
